(19) Europäisches Patentamt
European Patent Office
Office européen des brevets

(11) EP 0 665 234 B1

(12) EUROPÄISCHE PATENTSCHRIFT

(45) Veröffentlichungstag und Bekanntmachung des Hinweises auf die Patenterteilung:
**15.03.2000 Patentblatt 2000/11**

(51) Int. Cl.[7]: **C07F 7/30**, C07F 7/08, C07C 1/32, C07F 17/00

(21) Anmeldenummer: **94112301.0**

(22) Anmeldetag: **05.08.1994**

(54) **Verfahren zur Herstellung von Biscyclopentadienyl-Verbindungen**

Process for the preparation of biscyclopentadienyl compounds

Procédé de préparation de composés biscyclopentadiènyle

(84) Benannte Vertragsstaaten:
**AT BE CH DE ES FR GB GR IT LI NL PT SE**

(30) Priorität: **26.01.1994 DE 4402192**

(43) Veröffentlichungstag der Anmeldung:
**02.08.1995 Patentblatt 1995/31**

(73) Patentinhaber: **Witco GmbH**
**59192 Bergkamen (DE)**

(72) Erfinder: **Lisowsky, Dr. Richard**
**D-59174 Kamen (DE)**

(56) Entgegenhaltungen:
**EP-A- 0 376 154**

- **JOURNAL OF ORGANOMETALLIC CHEMISTRY, Bd.218, 1981 Seiten 159 - 167 SMITH, J.A. ET AL. 'ANSA-METALLOCENE DERIVATIVES III, INFLUENCE OF AN INTERANNULAR ETHYLENE BRIDGE ON THE REACTIVITY OF TITANOCENE DERIVATIVES'**
- **JOURNAL OF ORGANOMETALLIC CHEMISTRY, Bd.293, 1985 Seiten 271 - 283 DUFF, A.W. ET AL. '"DIBUTYLMAGNESIUM", A CONVENIENT REAGENT FOR THE SYNTHESIS OF USEFUL ORGANIC MAGNESIUM REAGENTS MGA2 INCLUDING CYCLOPENTADIENYLS, ARYLOXIDES, AND AMIDES. PREPARATION OF ZR(C5H5)CL3. X-RAY STRUCTURE OF (MG(.MU.-N(SIME)3)C6H4N (N(SIME3)-O)(OET2))2'**

EP 0 665 234 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die vorliegende Erfindung betrifft ein verbessertes Verfahren zur Herstellung von verbrückten Biscyclopentadienyl-Verbindungen.

Verbindungen dieser Art sind Ausgangskomponenten für z. B. chirale, stereorigide Metallocenverbindungen, welche in Kombination mit Co-Katalysatoren unter anderem als stereospezifische Katalysatorsysteme hoher Aktivität bei der Polymerisation von Olefinen eingesetzt werden (EP-A-0 351 392).

Es hat daher in der Vergangenheit eine Vielzahl von Vorschlägen gegeben, diese verbrückten Biscyclopentadienyl-Verbindungen herzustellen, wobei das allgemeine Reaktionsschema

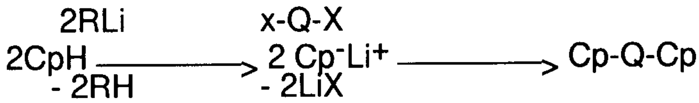


(CpH = Cyclopentadien, R = kurzkettiger Alkylrest, Q = Alkyl- oder Arylrest, X = Cl, Br, J, -O-Tosyl) weitgehend beibehalten wurde. Diese Verfahren haben den Nachteil, daß sie bei Temperaturen ≤ -70 °C und unter Mitverwendung von problematischen Lösungsmitteln wie Ethern, Methylenchlorid, Chloroform, Hexamethylenphosphorsäureamid (HMPA) arbeiten. Darüber hinaus war die Ausbeute an Biscyclopentadienyl-Verbindungen in der Regel gering und wurde durch erforderliche Reinigungsverfahren noch weiter verringert (Journal of Organometallic Chemistry, 1988, 342, 21-29, DE-OS 38 44 282).

Im Journal of Organometallic Chemistry, 218 (1981), 159-167 wird die Umsetzung von $(Cp)_2Mg$ mit Dibromethan in Diethylether unter Mitverwendung von Ethylendiamin bei Temperaturen von -78°C bis -70°C beschrieben.

Im Journal of Organometallic Chemistry, 293 (1985), 271-283 wird die Darstellung von Verbindungen (CpRa)2Mg durch Umsetzung von Dibutylmagnesium mit Cyclopentadiaryl-Derivaten in Heptan bei 0°C bis 20°C. Bei Inden und Fluoren versagte diese Methode unter den beschriebenen Bedingungen.

Aufgabe der vorliegenden Erfindung war es daher, ein Verfahren bereitzustellen, welches die Herstellung von verbrückten Biscyclopentadienyl-Verbindungen in wesentlich höheren Ausbeuten und hoher Reinheit ohne technisch aufwendige Verfahrensschritte ermöglicht.

Diese Aufgabe wurde überraschenderweise gelöst durch Umsetzung von Biscyclopentadienylmagnesium-Verbindungen $(CpRa)_2Mg$ mit geeigneten difunktionellen Verbindungen $XQX^1$.

Gegenstand der Erfindung ist daher ein Verfahren zur Herstellung von Biscyclopentadienyl-Verbindungen der allgemeinen Formel (1)

$$(CpRa)2\text{-}Q \qquad (1)$$

in welcher Cp ein- oder mehrfach substituierte Cyclopentadienylreste mit 1 ≤ a ≤4 oder Indenyl-Reste sein können, welche gegebenenfalls ein- oder mehrfach substituiert sein können, mit R = Alkyl-, Phosphin-, Amin-, Alkylether- oder Arylethergruppen mit 0 ≤ a ≤ 4 und Q eine ein- oder mehrgliedrige strukturelle Brücke zwischen den Cp-Ringen ist, mit Q =

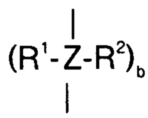

worin $R^1$ und $R^2$ gleich oder verschieden ein Wasserstoffatom, eine $C_1$-$C_{10}$-Alkylgruppe, $C_6$-$C_{10}$-Arylgruppe und Z Kohlenstoff, Silizium oder Germanium bedeutet und b = 1, 2, 3 sein kann, wobei in erster Stufe das entsprechende CpRa mit einer Magnesiumverbindung $(R^3R^4)_cMg$, worin $R^3$, $R^4$ unabhängig voneinander H, $C_1$-$C_{12}$-Alkylreste sein können und C = 0 oder 1 ist,unter Inertgasatmosphäre in einem inerten Lösungsmittel mit einem Siedepunkt ≥60 °C umgesetzt wird und in zweiter Stufe mit $QXX^1$, worin X, $X^1$ gleich oder verschieden Cl, Br, J, $-0\text{-}SO_2R^5$ mit $R^5$ = Alkylrest mit 1 - 10 C-Atomen oder Arylrest mit 6 - 10 C-Atomen sein kann, in dem inerten Lösungsmittel bei Raumtemperatur bis zum Siedepunkt des Lösungsmittels umgesetzt wird.

Die Verbindungen der allgemeinen Formel (1) werden hergestellt durch Umsetzung in erster Stufe nach der allgemeinen Gleichung

$$2CpRa + (R^3R^4)_cMg \rightarrow (CpRa)_2Mg + CR^3H + CR^4H$$

Hierin haben Cp, Ra, $R^3$, $R^4$, a, c die oben angegebene Bedeutung.

Die Umsetzungen erfolgen in Inertgasatmosphäre und unter Ausschluß von Sauerstoff und Feuchtigkeit. Hierbei werden erfindungsgemäß bevorzugt die Komponenten bei Raumtemperatur in einem inerten Lösungsmittel vorgelegt und die Temperatur unter intensivem Rühren erhöht.

Als inerte Lösungsmittel sind die auf diesem Gebiet üblichen mitverwendbar wie beispielsweise aliphatische oder cyclische Ether oder aromatische Kohlenwasserstoffe.

Erfindungsgemäß bevorzugt werden aliphatische Kohlenwasserstoffe mit Siedepunkten ≥60 °C, vorzugsweise ≥80 °C, insbesondere im Bereich von 80 - 120 °C. Die Umsetzung wird zur Erzielung praxisgerechter Reaktionszeiten vorzugsweise beim Siedepunkt der Lösungsmittel, insbesondere zwischen 80 - 120 °C, durchgeführt. Die Lösungsmittelmenge ist weitgehend unkritisch. Zur Erzielung hoher Raum-Zeit-Ausbeuten wird jedoch im oberen technisch möglichen Bereich gearbeitet.

Als Verbindungen $(R^3R^4)_cMg$ werden solche eingesetzt, in denen $R^3$ und $R^4$ gleich oder verschieden H, $C_{1-12}$-Alkylreste sind und C = 0 oder 1, vorzugsweise 1, ist. Erfindungsgemäß werden bevorzugt Butyl-Ethylmagnesium, Di-n-butylmagnesium, Di-n-hexylmagnesium, n-Butyl-sec-butylmagnesium in ihren handelsüblichen Formulierungen, wie insbesondere BOMAG®-A der Firma Witco GmbH (Butyl-octyl-magnesium 20 %ig in Heptan). In diesem Falle ist kein zusätzliches Lösungsmittel für Reaktionsführung erforderlich. Der Verlauf der Reaktion kann anhand der Gasentwicklung verfolgt werden.

Die so erhaltenen $(CpRa)_2Mg$-Verbindungen werden erfindungsgemäß bevorzugt direkt in zweiter Stufe mit den Verbindungen $X\text{-}Q\text{-}X^1$ nach der allgemeinen Reaktionsgleichung

$$(CpRa)_2Mg + XQX^1 \rightarrow (CpRa)_2Q + MgXX^1$$

zu den verbrückten Biscyclopentadienyl-Verbindungen umgesetzt.

Die zur Verbrückung einsetzbaren Komponenten $XQX^1$ sind die aus dem Stand der Technik bekannten Verbindungen (EP-A-0 480 390, EP-A-0 413 326, EP-A-0 530 908, EP-A-0 344 887, J. Chem. Soc. Dalton Trans., 1991, 2207). Erfindungsgemäß bevorzugt sind Verbindungen, in denen X, $X^1$ Cl, Br oder -O-Tosyl und Q = $-CH_2-CH_2-$; $-Si(CH_3)_2-$ sind.

Die Reaktionsmischung der ersten Stufe wird gegebenenfalls vor Zugabe der Komponente $XQX^1$ bis unter deren Siedetemperatur abgekühlt und nach erfolgter Zugabe erneut bis auf Siedetemperatur erwärmt.

Gegebenenfalls können zur Erhöhung der Reaktionsgeschwindigkeit noch in maximal stöchiometrischer Menge, bezogen auf Magnesium, Ether wie vorzugsweise Alkylether mit insbesondere 6 bis 10 C-Atomen wie insbesondere Di-n-butylether mitverwendet werden.

Die Reaktionszeiten liegen üblicherweise zwischen 1 bis 3 Stunden.

Bei dem erfindungsgemäßen Verfahren werden in beiden Stufen die Edukte vorzugsweise in stöchiometrischen Mengen eingesetzt. Dadurch und durch die nahezu quantitative Umsetzung unter praxisgrechten Bedingungen fallen die verbrückten Biscyclopentadienyl-Verbindungen in solchen Reinheiten an, daß sie direkt ohne Aufarbeitung für weitere Umsetzungen (wie beispielsweise zur Herstellung von Metallocenen) verwendbar sind.

Beispiele für die nach dem erfindungsgemäßen Verfahren herstellbaren verbrückten Biscyclopentadienyl-Verbindungen sind Dimethylsilyl-bis(1-inden), Dimethylsilyl-bis(1-cyclopentadien), 2,2-Propyl-bis(1-inden), 2,2-Propyl-bis(trimethylcyclopentadien), 2,2-Propyl-bis(5-dimethylamino-1-inden), 2,2-Propyl-bis(6-dipropylamino-1-inden), 2,2-Propyl-bis(4,7-bis(dimethyl-amino-1-inden), 2,2-Propyl-bis(5-diphenylphosphino-1-inden), 2,2-Propyl-bis(4,5,6,7-tetrahydro-1-inden), 2,2-Propyl-bis(4-methyl-1-inden), 2,2-Propyl-bis(5-methyl-1-inden), 2,2-Propyl-bis(6-methyl-1-inden), 2,2-Propyl-bis(7-methyl-1-inden), 2,2-Propyl-bis(5-methoxy-1-inden), 2,2-Propyl-bis(4,7-dimethoxy-1-inden), 2,2-Propyl-bis(2,3-dimethyl-1-inden), 2,2-Propyl-bis(4,7-dimethyl-1-inden),2,2-Propyl-bis(9-fluoren), 2,2-Propyl-bis(1-cyclopentadien), 2,2-Propyl-bis(1-inden), 2,2-Propyl-bis(1-inden)(1-cyclopentadien), 2,2-Propyl-bis(1-inden)(9-fluoren), Diphenylmethyl-bis(1-inden), Diphenylmethyl-bis(9-fluoren), Diphenylmethyl-bis(1-cyclopentadien), Diphenylmethyl-bis(1-inden), Diphenylmethyl-bis(1-inden)(1-cyclopentadien), Diphenylsilyl-bis(1-iden), Diphenylsilyl-bis(1-cyclopentadien), Diphenylsilyl-bis(1-inden), Diphenylsilyl-bis(1-inden)(1-cyclopentadien), Ethylen-bis(1-inden), Ethylen-bis(trimethylcyclo-pentadien), Ethylen-bis(5-dimethylamino-1-inden), Ethylen-bis(6-dipropylamino-1-inden), Ethylen-bis(4,7-bis(dimethylamino)-1-inden), Ethylen-bis(5-diphenylphosphino-1-inden), Ethylen-bis(4,5,6,7-tetrahydro-1-inden), Ethylen-bis(4-methyl-1-inden), Ethylen-bis(5-methyl-1-inden), Ethylen-bis(6-methyl-1-inden), Ethylen-bis(7-methyl-1-inden), Ethylen-bis(5-methoxy-1-inden), Ethylen-bis(4,7-dimethoxy-1-inden), Ethylen-bis(2,3-dimethyl-1-inden), Ethylen-bis(4,7-dimethyl-1-inden), Ethylen-bis(1-cyclopentadien), Ethylen-bis(1-inden).

Beispiele

Alle Versuche werden unter Ausschluß von Sauerstoff und Feuchtigkeit unter Stickstoff als Inertgas durchgeführt.

Beispiel 1

Darstellung von Ethylen-bis(inden-1-yl)$_2$:

Es wird bei Raumtemperatur eine Mischung von 812 ml BOMAG-A® (0,71 mol; Butyloctylmagnesium der Firma Witco GmbH; 20 %ig in Heptan) und 184 ml Inden (90 %ig; 1,42 mol) hergestellt, indem in einem 2 l-Glaskolben das Butyloctylmagnesium vorgelegt und das Inden zugefügt wird. Anschließend wird 3 h unter Rückfluß reagieren gelassen, bis das Aufhören der Gasentwicklung das Reaktionsende anzeigt.
Nach Abkühlen auf 70 °C werden 61,2 ml (0,71 mol) 1,2-Dibromethan und 100 ml Di-n-butylether als Gemisch über einen Tropftrichter zudosiert.
Erneut wird 2 h refluxiert, wonach eine GC-Kontrolle der Reaktionslösung quantitativen Umsatz anzeigt bei Bildung von 96 % EthylenIndenyl$_2$.
Nach Abkühlen auf Raumtemperatur wird das ausgefallene $MgBr_2$ mittels Filtration abgetrennt und das Filtrat zur Trockene eingeengt und aus Methanol umkristallisiert.
Man erhält 167 g an EthylIndenyl$_2$ (91 % d. Th)

$^1$H-NMR ($CDCl_3$): (Isomerengemisch)

Isomer I: 7,5 - 7,1 (m, 8 H); 6,87 (d, 2 H); 6,57 (d, 2 H); 3,5 (m (b), 2 H); 2,1 - 1,5 (m, 8 H)
Isomer II: 7,6 - 7,2 (m, 8 H); 6,35 (s, 2 H); 3,4 (s, 4 H); 3,0 (s, 4 H).

Beispiel 2

Darstellung von Ethylen-bis(inden-1-yl)$_2$:

a) ohne Einsatz von Di-n-butylether

Es werden 416 ml BOMAG-A® (20 %ig in Heptan; 0,364 mol) in einem 1-l-Glaskolben vorgelegt und auf Rückfluß erhitzt. Dann werden innerhalb vom 30 min 85,2 ml Inden (94 %ig; 0,73 mol) über einen Tropftrichter zugegeben.
Es wird 6 h refluxiert.
Bei 60 - 70 °C werden 31,4 ml 1,2-Dibromethan (0,364 mol) ohne Zusatz von Di-n-butylether zugegeben, wodurch sich die Reaktionszeit verlängert, so daß man 5 h unter Rückfluß bis zum vollständigen Umsatz nachreagieren lassen muß.
Nach Aufarbeitung wie in Beispiel 1 erhält man in 85 %iger Ausbeute Ethylen-bis-(inden-1-yl) (79,9 g).

b) Unter Einsatz von Ethylenglycoldi-(p-toluolsulfonat):

10 g Inden (95 %ig; 82 mmol) werden bei Raumtemperatur mit 34,2 g BOMAG-A® (20 %ig in Heptan; 41 mmol) versetzt und 4 h lang unter Rückfluß erhitzt.
Dann werden bei Raumtemperatur Ethylenglycoldi-(p-toluolsulfonat) (15,7 g; 97 %ig; 41 mmol) und 5,3 g (41 mmol) Di-n-butylether zugegeben und erneut 1,5 h unter Rückfluß gerührt. Man erhält nach Aufarbeitung in 89 %iger Ausbeute Ethylen-bis(inden-1-yl) (18,9 g).

Beispiel 3

Darstellung von Bis(inden-1-yl)dimethylsilan:

49,6 ml Inden (95 %ig; 0,404 mol) und 50 ml Heptan werden in einem 500 ml Kolben vorgelegt und innerhalb von 15 min unter Rückfluß mit 231 ml BOMAG-A® (20 %ig in Heptan; 0,202 mol) versetzt.
Nach 3 h Rückfluß wird auf Raumtemperatur abgekühlt.
Man dosiert weiterhin ein Gemisch von 26,1 g $Me_2SiCl_2$ (0,202 mol), 35 ml Di-n-butylether und 20 ml Hexan über einen Tropftrichter zur Reaktionslösung und refluxiert 2h.
GC-Kontrolle des Ansatzes zeigt quantitativen Umsatz bei Bildung von 94 % $Me_2$SiIndenyl$_2$ an.
Filtration, Entfernen des Lösungsmittels und Umkristallisation aus Methanol liefern 50,2 g (86 % d. Th.) an Bis(inden-1-yl)dimethylsilan.

$^1$H-NMR ($CDCl_3$): (Isomerengemisch)

| | |
|---|---|
| Isomer I.): | 7,5 - 7,0 (m, 8H); 6,75 (d, 2H); 6,70 (d, 2H); 3,7 (s, 4 H), -0,2 (s, 6H) |
| Isomer II.): | 7,5 - 7,0 (m, 8H); 6,55 (d, 2H); 6,52 (d, 2H); 3,7 (s, 4 H), 0,05 (s, 3H); -0,4 (s, 3H) |

Beispiel 4

Einsatz weiterer Dialkymagnesiumverbindungen:

a) Beispiel 3 wird wiederholt; anstelle von BOMAG-A® wird jedoch Dibutylmagnesium (1 molar in Heptan) eingesetzt. Dabei werden die Refluxionszeiten bei der Umsetzung des Dialkylmagnesiums jeweils um 30 min verlängert.

Es können vergleichbare Ausbeuten an Bis(inden-1-yl)dimethylsilan erzielt werden (83 %; 48,4 g).

b) Ansatz 4 a) wird mit Dihexylmagnesium (1 molar in Heptan) durchgeführt.

Auch hier werden vergleichbare Ausbeuten erzielt (87 %; 50,8 g)

Vergleichsbeispiel 5

Darstellung von $Me_2Si(indenyl)_2$ via Li, Na-Derivat des Indens:

Wird die Verbindung $Me_2SiIndenyl_2$ über IndenylLi oder IndenylNa hergestellt, so findet man je nach Reaktionsbedingung ein- bis mehrfache Überalkylierung.

a) $Me_2SiCl_2$ (55,97 g; 0,43 mol) werden in 85 ml Diethylether vorgelegt. Anschließend wird bei -70 °C über einen Zeitraum von 4 h IndenylLi (105 g; 0,86 mol) in 600 ml Diethylether zugegeben.

Nach beendeter Zugabe wird die Kühlung entfernt und auf Raumtemperatur (RT) kommen gelassen. Es wird 8 h bei RT nachgerührt.

Die Reaktionslösung wird mittels Gaschromatographie (GC), bzw. Gaschromatographie-Massenspektroskopioe (GC-MS)-Kopplung analysiert und enthält:

| | | |
|---|---|---|
| I.) | $Me_2SiIndenyl_2$: | 95 % |
| II.) | $Me_2SiIndenyl_2(Me_2SiIndenyl)$: | 5 % |
| III.) | $Me_2SiIndenyl_2(Me_2SiIndenyl)(Me_2SiIndenyl)$: | - |

Es können 75 % d. Th an reinem Produkt isoliert werden.

b) Analoge Durchführung wie in a) jedoch bei 20 °C ergibt:

| | |
|---|---|
| I.) | 90 % |
| II.) | 10 % |
| III.) | 0,2 % |

c) 56,8 g (0,44 mol) $Me_2SiCl_2$ werden in 100 ml Diethylether vorgelegt und bei 0 °C mit einer Lösung von 121,6 g IndenylNa (0,88 mol) in 400 ml THF innerhalb von 1,5 h versetzt.

Die Untersuchung des Reaktionsgemisches ergibt:

| | |
|---|---|
| I.) | 80 % |
| II.) | 17 % |

(fortgesetzt)

| | |
|---|---|
| III.) | 2 % |

Nach Aufarbeitung und Kristallisation können 50 % d. Th. an reinem $Me_2SiIndenyl_2$ isoliert werden.

d) Als Vergleich dazu wird ein Ansatz nach dem erfindungsgemäßen Verfahren durchgeführt:
Zu einer Suspension von $Indenyl_2Mg$ (102 g; 0,4 mol)in 300ml Heptan werden 0,4 mol $Me_2SiCl_2$ (51,6 g), gelöst in 50 ml Hexan und versetzt mit 0,4 mol n-$Butyl_2O$, bei RT gegeben.

Anschließend wird sofort auf Rückfluß erhitzt und 3 h dabei belassen.

Analyse der Reaktionslösung :

| | |
|---|---|
| I.) | 99 % |
| II.) | <0,2 % |
| III.) | - |

Aufarbeitung und Kristallisation liefern durchschnittliche Ausbeuten von >85 % an $Me_2Indenyl_2$.

Setzt man die Verbindung ohne Isolierung weiter ein, so ist, bedingt durch die nicht vorhandenen Aufarbeitungsverluste, die Ausbeute nahezu quantitativ.

Beispiel 6

Darstellung von Bis(inden-1-yl)dimethylgerman:

24 g BOMAG-A® (20 %ig; 28,8 mmol) werden in einem 100 ml Glaskolben vorgelegt und auf Rückfluß erhitzt. Dann erfolgt die Zugabe von 7,4 g Inden (90 %ig; 57,6 mmol) mit anschließendem vierstündigem Refluxieren.

Bei Raumtemperatur wird ein Gemisch bestehend aus 5 g $Me_2GeCl_2$ (28,8 mmol) und 3,7 g Di-n-butylether hinzugefügt. 2 h Refluxieren schließen sich an.

Nach Aufarbeitung können 7,9 g $Me_2GeIndenyl_2$ (83 % d. Th.) isoliert werden.

Beispiel 7

Darstellung von $Me_2Si(1{,}3butylmethylCp)_2$:

20 g 1-Butyl-3-methyl-cyclopentadien (0,147 mmol) werden bei Raumtemperatur mit 84 ml BOMAG-A® (20 %ig in Heptan; 73,4 mmol) versetzt.

Nach 3 h Rückfluß wird auf RT abgekühlt.

Man dosiert weiterhin 9,5 g $Me_2SiCl_2$ (73,4 mmol), 12,7 ml Di-n-butylether und 7 ml Hexan zur Reaktionslösung und refluxiert 6 h. GC-Kontrolle des Ansatzes zeigt quantitativen Umsatz bei Bildung von 85 % $Me_2Si(1{,}3\text{-butylmethylCp})_2$.

Filtration, Lösungsmittelentfernen und Kugelrohrdestillation liefern 16,8 g (70 % d. Th.) an $Me_2Si(\text{butylmethylCp})_2$.
(Molmasse mittels GC-MS ermittelt: 328)

$^1H$-NMR ($CDCl_3$): (Isomerengemisch)
6,2 - 5,8 (m, H-C=C-); 3,3 - 2,9 (m, H-C-C=C-); 2,5 - 2,3 (m, $-CH_2-$); 2,15 - 1,95 (m; $-CH_3$); 1,6 - 1,25 (m, $-CH_2CH_2-$); 1,0 - 0,85 (m, $-CH_3$); 0,15 - -0,25 (m, $H_3C$-Si).

**Patentansprüche**

1. Verfahren zur Herstellung von Biscyclopentadienyl-Verbindungen der allgemeinen Formel (1)

(CpRa)2-Q (1)

in welcher Cp ein- oder mehrfach substituierte Cyclopentadienylreste mit $1 \leq a \leq 4$ oder Indenyl-Reste sein können, welche gegebenenfalls ein- oder mehrfach substituiert sein können, mit R = Alkyl-, Phosphin-, Amin-, Alkylether- oder Arylethergruppen mit $0 \leq a \leq 4$ und Q eine ein- oder mehrgliedrige strukturelle Brücke zwischen den Cp-Ringen ist, mit Q =

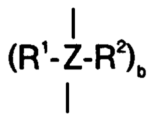

worin $R^1$ und $R^2$ gleich oder verschieden ein Wasserstoffatom, eine $C_1$-$C_{10}$-Alkylgruppe, $C_6$-$C_{10}$-Arylgruppe und Z Kohlenstoff, Silizium oder Germanium bedeutet und b = 1, 2, 3 sein kann, wobei in erster Stufe das entsprechende CpRa mit einer Magnesiumverbindung $(R^3R^4)_cMg$, worin $R^3$, $R^4$ unabhangig voneinander H, $C_1$-$C_{12}$-Alkylreste sein können und C = 0 oder 1 ist, unter Inertgasatmosphäre in einem inerten Lösungsmittel mit einem Siedepunkt ≥60 °C umgesetzt wird und in zweiter Stufe mit $QXX^1$, worin X, $X^1$ gleich oder verschieden Cl, Br, J, $-0-SO_2R^5$ mit $R^5$ = Alkylrest mit 1 - 10 C-Atomen oder Arylrest mit 6 - 10 C-Atomen sein kann, in dem inerten Lösungsmittel bei Raumtemperatur bis zum Siedepunkt des Lösungsmittels umgesetzt wird.

**2.** Verfahren gemäß Anspruch 1, dadurch gekennzeichnet, daß die Gruppe

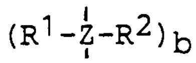

ein Ethylenrest oder eine $-R^1-SiR^2$-Gruppe ist, worin $R^1$, $R^2$ gleich oder verschieden $CH_3$, $C_2H_5^-$Phenyl-Reste sein können.

**3.** Verfahren zur Herstellung von Biscyclopentadienyl-Verbindungen gemäß Anspruch 1, dadurch gekennzeichnet, daß als Lösungsmittel Petrolether, Heptan, Oktan, Toluol, Xylol verwendet werden.

**4.** Verfahren zur Herstellung von Biscyclopentadienyl-Verbindungen gemäß Anspruch 1, dadurch gekennzeichnet, daß als $(R^3R4)_cMg$ Verbindungen, in denen c = 1 und $R^3$, $R^4$ gleich oder verschieden $C_2H_5$-, $C_4H_9$, $C_6H_{13}$-, $C_8H_{17}$ sein können oder deren Isomeren allein oder in Mischung miteinander oder untereinander verwendet werden.

**5.** Verfahren zur Herstellung von Biscyclopentadienyl-Verbindungen gemäß Anspruch 4, dadurch gekennzeichnet, daß als $(R^3R^4)_cMg$ eine Verbindung mit $R^3$ = n-Butyl, $R^4$ = n-Octyl mit $R^3 : R^4 = 3 : 1$ und c = 1 verwendet wird.

**6.** Verfahren zur Herstellung von Bicyclopentadienylverbindungen gemäß Anspruch 1, dadurch gekennzeichnet, daß in zweiter Stufe Ether in maximal stöchiometrischer Menge, bezogen auf Magnesium, mitverwendet werden.

## Claims

**1.** Process for preparing biscyclopentadienyl compounds of the general formula (1)

$$(CpRa)_2\text{-}Q \qquad (1)$$

in which Cp can be singly or multiply substituted cyclopentadienyl radicals with $1 \leq a \leq 4$ or indenyl radicals which can, if desired, be singly or multiply substituted, R = alkyl, phosphine, amine, alkyl ether or aryl ether groups with $0 \leq a \leq 4$, and Q is a single-membered or multi-membered structural bridge between the Cp rings, where Q =

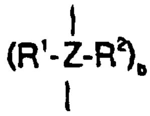

where $R^1$ and $R^2$ are identical or different and are a hydrogen atom, a $C_1$-$C_{10}$-alkyl group, $C_6$-$C_{10}$-aryl group and Z is carbon, silicon or germanium and b can be 1, 2, 3, wherein, in a first step, the corresponding CpRa is reacted

with a magnesium compound $(R^3R^4)_cMg$, where $R^3$, $R^4$ can be, independently of one another, H, $C_1$-$C_{12}$-alkyl radicals and c = 0 or 1, under an inert gas atmosphere in an inert solvent having a boiling point ≥ 60°C, and, in a second step, with $QXX^1$, where X, $X^1$ can be identical or different and are Cl, Br, I, -O-$SO_2R^5$ with $R^5$ = an alkyl radical having 1 - 10 carbon atoms or an aryl radical having 6 - 10 carbon atoms, in the inert solvent at from room temperature to the boiling point of the solvent.

**2.** Process according to Claim 1, characterized in that the group

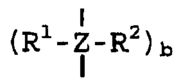

is an ethylene radical or an - $R^1$-Si$R^2$- group where $R^1$, $R^2$ can be identical or different and can be $CH_3$, $C_2H_5$-phenyl radicals.

**3.** Process for preparing biscyclopentadienyl compounds according to Claim 1, characterized in that petroleum ether, heptane, octane, toluene, xylene are used as solvent.

**4.** Process for preparing biscyclopentadienyl compounds according to Claim 1, characterized in that $(R^3R^4)_cMg$ compounds, in which c = 1 and $R^3$, $R^4$ can be identical or different and can be $C_2H_5$-, $C_4H_9$, $C_6H_{13}$-, $C_8H_{17}$ or isomers thereof, are used alone or in admixture with one another or among one another.

**5.** Process for preparing biscyclopentadienyl compounds according to Claim 4, characterized in that the $(R^3R^4)_cMg$ used is a compound with $R^3$ = n-butyl, $R^4$ = n-octyl with $R^3 : R^4 = 3 : 1$ and c = 1.

**6.** Process for preparing bicyclopentadienyl compounds according to Claim 1, characterized in that, in a second step, ethers are also used in at most the stoichiometric amount, based on magnesium.

**Revendications**

**1.** Procédé de préparation de composés biscyclopentadiényle de formule générale (1)

$$(CpRa)_2\text{-}Q \qquad (1)$$

dans laquelle Cp peuvent être des radicaux cyclopentadiènyle une ou plusieurs fois substitués avec 1≤a≤4 ou des radicaux indényle, lesquels peuvent le cas échéant être substitués une ou plusieurs fois, par R = groupements alkyle, phosphine, amine, alkyléther ou aryléther avec 0≤a≤4 et Q est un pont structurel à un ou plusieurs membres entre les cycles Cp, avec Q =

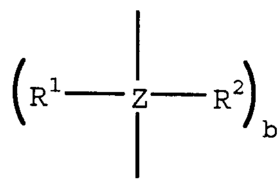

dans laquelle $R^1$ et $R^2$, identiques ou différents, signifient un atome d'hydrogène, un groupement alkyle en $C_1$-$C_{10}$, un groupement aryle en $C_6$-$C_{10}$ et Z, du carbone, du silicium ou du germanium et b peut être = 1, 2, ou 3, le CpRa correspondant étant transformé dans la première étape avec un composé de magnésium $(R^3R^4)_cMg$, dans lequel $R^3$, $R^4$ indépendamment l'un de l'autre peuvent être des radicaux H, alkyle en $C_1$-$C_{12}$ et C = 0 ou 1, sous une atmosphère de gaz inerte dans un solvant inerte avec un point d'ébullition ≥ 60°C et étant transformé dans une deuxième étape avec du $QXX^1$, dans lequel X, $X^1$, identiques ou différents, peuvent être du Cl, du Br, de l'I, -0-$SO_2R^5$ avec $R^5$ = un radical alkyle avec 1-10 atomes de carbone ou un radical aryle avec 6-10 atomes de carbone, dans le solvant inerte à température ambiante jusqu'au point d'ébullition du solvant.

**2.** Procédé suivant la revendication 1, caractérisé en ce que le groupement

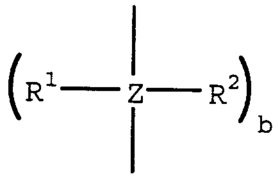

est un radical éthylène ou un groupement $-R^1-SiR^2-$, dans lequel $R^1$, $R^2$ peuvent être identiques ou différents, un radical $CH_3$, un radical $C_2H_5$, un radical phényle.

3. Procédé de préparation de composés biscyclopentadiényle suivant la revendication 1, caractérisé en ce qu'on utilise comme solvant de l'éther de pétrole, de l'heptane, de l'octane, du toluène, du xylène.

4. Procédé de préparation de composés biscyclopentadiényle suivant la revendication 1, caractérisé en ce qu'on utilise comme composés $(R^3R^4)_cMg$, dans lesquels c = 1 et $R^3$, $R^4$ peuvent être, identiques ou différents, $C_2H_5$-, $c_4H_9$-, $C_6H_{13}$-, $C_8H_{17}$- ou leurs isomères seuls ou en mélange les uns avec les autres ou entre eux.

5. Procédé de préparation de composés biscyclopentadiényle suivant la revendication 4, caractérisé en ce qu'on utilise comme $(R^3R^4)_cMg$, un composé avec $R^3$ = n-butyle, $R^4$ = n-octyle avec $R^3:R^4$ = 3:1 et c = 1.

6. Procédé de préparation de composés biscyclopentadiényle suivant la revendication 1, caractérisé en ce qu'on utilise conjointement dans la deuxième étape de l'éther en quantité stoechiométrique maximale sur base du magnésium.